**Europäisches Patentamt**

**European Patent Office**

**Office européen des brevets**

(19)

(11) Veröffentlichungsnummer: **0 133 252**
A1

# EUROPÄISCHE PATENTANMELDUNG

(12)

(21) Anmeldenummer: 84108566.5

(22) Anmeldetag: 19.07.84

(51) Int. Cl.⁴: **A 61 K 9/72**, A 61 K 31/55

(30) Priorität: 20.07.83 DE 3326089

(43) Veröffentlichungstag der Anmeldung: 20.02.85
Patentblatt 85/8

(84) Benannte Vertragsstaaten: **AT BE CH DE FR GB IT LI LU NL SE**

(71) Anmelder: **GÖDECKE AKTIENGESELLSCHAFT, Salzufer 16, D-1000 Berlin 10 (DE)**

(72) Erfinder: **Fritschi, Edgar, Dr., Am Scheuerwald 2, D-7811 St. Peter (DE)**

(54) Zur Inhalation bestimmte Darreichungsform von Calcium-Antagonisten.

(57) Die Anmeldung betrifft zur Inhalation bestimmte therapeutische Darreichungsformen, die dadurch gekennzeichnet sind, daß als Wirkstoffe Calcium-Antagonisten eingesetzt werden. Es werden Dosier-Aerosole von Ca-Antagonisten insbesondere von Diltiazem, sowie Verfahren zu deren Herstellung beschrieben.

EP 0 133 252 A1

ACTORUM AG

Beschreibung

Die langfristige Therapie der Angina pectoris wird heute übligcherweise mit Nitraten, wie z.B. Isosorbid-dinitrat oder Isosorbid-mononitrat oder aber mit Calcium-Antagonisten durchgeführt.

Erheblich schwieriger ist die Kupierung des akuten Angina-pectoris-Anfalls, da die genannten Wirkstoffe einen sofortigen Wirkungseintritt nur bei intravenöser Verabreichung ermöglichen. Diese kann aber nur durch den Arzt vorgenommen werden, der in den meisten Fällen nicht zur Stelle ist, wenn sich der Angina-pectoris-Anfall ereignet. Ein Nachteil der parenteralen Behandlung ist auch, daß es im Fall der Calcium-Antagonisten aufgrund starker peripherer Nebenwirkungen bei rascher Resorption leicht zu orthostatistischen Zuständen kommt.

Als praktikabler Ausweg der Anfallsbehandlung durch den Patienten bietet sich derzeit lediglich die sublinguale oder inhalative Verabreichung von Nitroglycerin, da dieser Wirkstoff aufgrund seiner physikalisch-chemischen Eigenschaften und seines speziellen pharmakologischen Profils sofort wirksam sein kann.

Da der Fachmann in Kenntnis der starken peripheren Wirkung von Calcium-Antagonisten die Sofortwirkung dieser Wirkstoffklasse therapeutisch bisher nicht ausnutzte, blieb zur Behandlung des akuten Anfalls praktisch nur Nitroglycerin übrig, wobei aber auch hier Nebenwirkungen, wie Nausea, Schwindel, Tachykardie und plötzlicher Blutdruckabfall hingenommen werden müssen.

Es wurde nun überraschend gefunden, daß Calcium-Antagonisten bei inhalativer Verabreichung sofort wirksam sind und eine sofortige Vermehrung des Blutflusses in der Koronararterie bewirken, ohne jedoch die Peripherie ungünstig zu beeinflussen.

Calcium-Antagonisten eignen sich daher unerwartet auch zur Therapie des Angina-pectoris-Anfalls, wenn sie inhalativ verabreicht werden. Sie bieten gegenüber der bisher bekannten Therapie den großen Vorteil, daß die beschriebenen Nebenwirkungen des Nitroglycerins vermieden werden und die calciumantagonistische Aktivität, die bekanntlich das pathophysiologische Geschehen am sinnvollsten beeinflußt, auch im akuten Anfall ausgenutzt werden kann.

Gegenstand der vorliegenden Erfindung ist somit eine zur Inhalation bestimmte therapeutische Darreichungsform enthaltend gegebenenfalls inhalierbare Treib- und/oder Trägerstoffe und mindestens einen Wirkstoff zur Behandlung der Angina pectoris, dadurch gekennzeichnet, daß der Wirkstoff ein Calcium-Antagonist ist.

Ein weiterer Gegenstand der Erfindung ist ein Verfahren zur Herstellung eines sofort wirksamen Arzneimittels zur Behandlung des Angina-pectoris-Anfalls, das dadurch gekennzeichnet ist, daß man als Wirkstoff einen Calcium-Antagonisten in fester oder gelöster Form in einen inhalierbaren Treib- und/oder Trägerstoff einbringt und den Treib- und/oder Trägerstoff zusammen mit dem Wirkstoff unter Druck oder durch Kühlung unter den Siedepunkt des Treib- oder Trägerstoffs in ein zur Inhalation der so hergestellten Arzneimittelzubereitung geeignetes Inhalationsgefäß abfüllt und dieses gasdicht verschließt.

Ein weiterer Gegenstand der Erfindung ist die Verwendung von Calcium-Antagonisten bei der inhalativen Bekämpfung der Angina-pectoris.

Bekannte Calciumantagonisten sind z.B. Nifedipin, Verapamil, Etafenon, Prenylamin, Perhexilin und Gallopamil. Im Rahmen der vorliegenden Erfindung wird Diltiazem als für die neue Therapieform besonders geeignet bevorzugt.

Die Wirkstoffe können, sofern sie wie z.B. Diltiazem ·HCl wasserlöslich sind, in eine wässrige Lösung gebracht und mit Stickstoff gegebenenfalls unter Druck in einen Spraybehälter abgefüllt werden.

Üblicher ist es jedoch, den Wirkstoff zusammen mit einem Suspendierhilfsmittel, wie z.B. Sorbitantrioleat bei etwa 5 bis 20°C mit einem bei dieser Temperatur flüssigen Fluorkohlenwasserstoff zu mischen, im Kaltabfüllverfahren diese Mischung zusammen mit dem Sicherheitstreibmittelgemisch (FKW: 12:114) in den Spraybehälter zu geben und diesen sofort zu verschließen.

Eine dritte Möglichkeit besteht darin, den Wirkstoff in mikronisierter Form, d.h. mit einem Partikeldurchmesser zwischen 0,5 und 5 µm, zusammen mit einem entsprechend feinteiligen inhalierbaren Trägerstoff wie z.B. Milchzucker in eine Hartgelatinekapsel zu füllen und die therapeutische Anwendung mittels eines üblichen mechanischen Pulverinhalators durchzuführen.

Die Dosierung der Inhalatoren sollte so eingestellt werden, daß eine Einmaldosis für erwachsene Menschen zwischen 5 und 50 mg liegt.

Die folgenden Beispiele dienen der näheren Erläuterung der Erfindung:

- 4 -

BEISPIEL 1

1,632 kg Diltiazem · HCl (mikronisiert) werden mit 500 g Sorbitantrioleat und 37,42 kg Freon ® 11, bei einer Temperatur von ca. 10 °C gemischt und desagglomerierend suspendiert. Die so erhaltene Suspension wird dann in 170,45 kg Sicherheitstreibmittelgemisch (FKW 12:114) bei einer Temperatur von - 50 °C eingetragen und das Gemisch homogenisiert. Anschließend wird in Aluminiumdosen kalt abgefüllt, die sofort mit einem Dosierventil verschlossen werden.

Man erhält so 10.000 Dosen (Nutzinhalt 22 ml) für 300 Hübe à 250 µl entsprechend 0,544 mg Diltiazem · HCl Wirkstoff/Hub.

Beispiel 2

544 g Diltiazem · HCl (mikronisiert) werden mit 4,456 kg Lactose (mikronisiert) homogen vermischt und in Hartgelatinekapseln mit einem Wirkstoffgehalt von 5,44 mg Diltiazem HCl (entspr. 5 mg Base) abgefüllt.

Die Kapseln können mittels eines Pulverinhalators perforiert werden, so daß der Kapselinhalt inhaliert werden kann.

Zum Wirksamkeitsnachweis der neuen Darreichungsform wurden folgende Versuche durchgeführt.

Versuchstiere waren 2 Bastardhunde (♀, Körpergewicht 16 kg bzw. 18 kg) in Pentobarbitalnarkose (30 mg/kg i.v. initial). Die Tiere waren tracheotomiert und wurden über eine ypsilonförmige Trachealkanüle, deren einer Schenkel zur Substanzinsufflation diente, mit einem Engström-Respirator (Typ ER 300) mit Raumluft beatmet.

Folgende Parameter der Tiere wurden gemessen.

1. EKG-Extremitätenleitung II
2. Herzfrequenz (R-Zacken-getriggert vom EKG) mit einem Pulsfrequenzmesser (in mmHg)
3. Arterieller Blutdruck in der rechten Femoralarterie mit einem Tipkatheter (in mmHg)
4. Der linksventrikuläre Druck des Herzens mit einem Tipkatheter via rechte arteria carotis (in mmHg)

5. Die Kontraktilität des Herzens mit einem HSE-Differenzierer aus dem isometrischen Anteil der linksventrikulären Druckkurve, differenziert als $dp/dt_{max}$ (in mmHg/sec)
6. Die Blutströmung in der arteria femoralis sinistra mittels elektromagnetischer Flußmessung, (in ml/min)

7. Die Blutströmung in der linken arteria coronaria descendens

Alle Meßwerte wurden fortlaufend simultan mit einem Direktschreiber aufgezeichnet und jeweils 1; 5 und 10 min nach Applikation ausgemessen und ausgedruckt.

- 6 -

Die Applikation der Substanz erfolgte mit einem pistolenähnlichen Druckluftdosierer. Die gewichtsbezogene Menge der Ursubstanz wurde von vorne in das Ausblasrohr des Dosierers eingefüllt und während der pumpengesteuerten Inspiration mit einem Druck von 0,2 atü vollständig in die Trachea des Tieres eingeblasen. Der Einblasvorgang dauerte jeweils 1 sec. und hatte allein, wie in Vorversuchen gefunden wurde, keinerlei Einfluß auf die gemessenen Kreislaufgrößen.

Die Ergebnisse der Versuche sind in der nachfolgenden Figur I graphisch dargestellt. Den Kurven ist zu entnehmen, daß weder die Herzfrequenz noch die Kontraktilität beeinflußt wird, daß aber der Fluß in der Koronararterie dosisabhängig, sofort und langanhaltend zunimmt.

Weiterhin ist ersichtlich, daß der arterielle Blutdruck erst nach hohen Dosen und auch dann erst deutlich nach der Beeinflussung des Koronarflusses geringfügig abnimmt.

0133252

- 1 -

1.) Zur Inhalation bestimmte therapeutische Darreichungsform
enthaltend gegebenenfalls inhalierbare Treib- und/oder Trägerstoffe und mindestens einen
Wirkstoff zur Behandlung der Angina pectoris, dadurch
gekennzeichnet, daß der Wirkstoff ein Calcium-Antagonist
ist.

2.) Darreichungsform gemäß Anspruch 1, dadurch gekennzeichnet, daß der Wirkstoff Diltiazem oder ein pharmakologisch unbedenkliches Salz desselben ist.

II. Patentansprüche für AT

1.) Verfahren zur Herstellung eines sofort wirksamen Arzneimittels zur Behandlung des Angina-pectoris Anfalls, dadurch gekennzeichnet, daß man als Wirkstoff einen Calcium-Antagonisten in fester oder gelöster Form in einen inhalierbaren Treib- und/oder Trägerstoff einbringt und den Treib- und/oder Trägerstoff zusammen mit dem Wirkstoff unter Druck oder durch Kühlung unter den Siedepunkt des Treib- oder Trägerstoffs in ein zur Inhalation der so hergestellten Arzneimittelzubereitung geeignetes Inhalationsgefäß abfüllt und dieses gasdicht verschließt.

2.) Verfahren nach Anspruch 1, dadurch gekennzeichnet, daß als Calcium-Antagonist Diltiazem verwendet wird.

Figur I

Messung verschiedener Kreislaufparameter an narkotisierten Hunden nach Einblasen von Dilzem in die Bronchien. (n = 2)

0133252

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A- 970 027 (REVLON, INC.) * Seite 1, Zeile 69 - Seite 2, Zeile 62 * | 1-2 | A 61 K 9/72 A 61 K 31/55 |
| A | US-A-4 175 128 (COLIN R. TAYLOR) * Spalte 8, Zeilen 5-16 (Beispiel 7), Zeilen 41-64 (Ansprüche 1-5) * | 1-2 | |

RECHERCHIERTE SACHGEBIETE (Int. Cl.4)

A 61 K

Der vorliegende Recherchenbericht wurde für alle Patentansprüche erstellt.

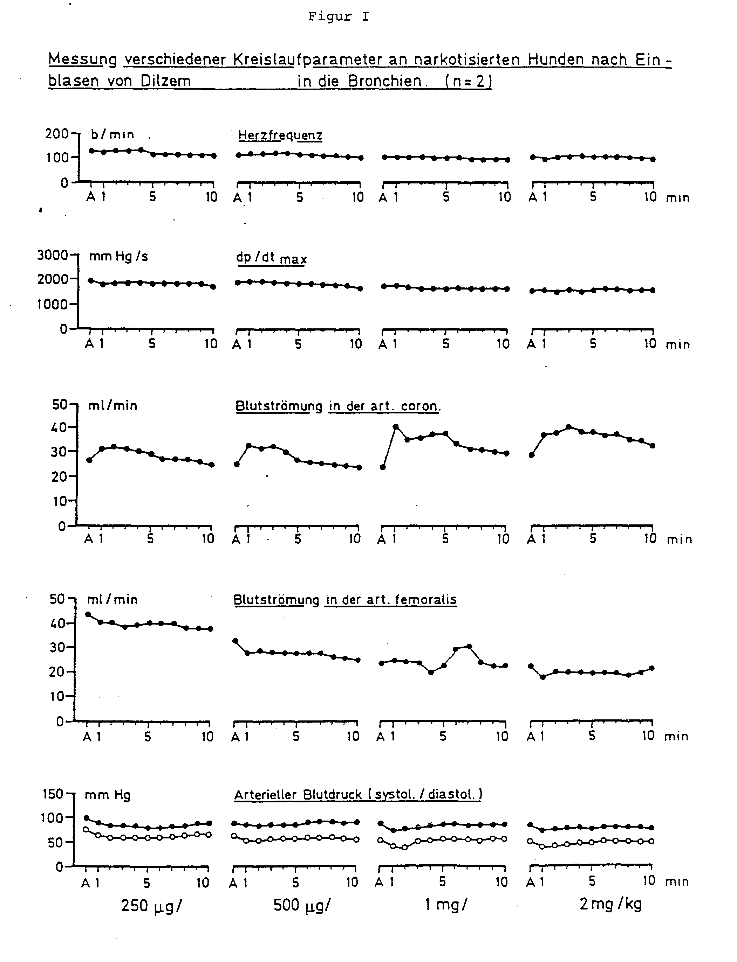

| Recherchenort | Abschlußdatum der Recherche | Prüfer |
|---|---|---|
| DEN HAAG | 23-10-1984 | BRINKMANN C. |